# EUROPÄISCHE PATENTSCHRIFT

(11) **EP 0 721 950 B1**
(45) Veröffentlichungstag und Bekanntmachung des Hinweises auf die Patenterteilung: **06.05.1998**
(21) Anmeldenummer: 96100093.2
(22) Anmeldetag: 05.01.1996
(51) Int. Cl.: C07D 417/04, C07D 417/14, A61K 31/54

(54) **Kardioaktive 3-Alkoxycarbonyl-thiadiazinone**
Cardioactive 3-alkoxycarbonyl-thiadiazinones
3-Alkoxycarbonyl-thiadiazinones cardioactives

(30) Priorität: 11.01.1995 DE 19500558
(43) Veröffentlichungstag der Anmeldung: 17.07.1996
(73) Patentinhaber: MERCK PATENT GmbH, 64293 Darmstadt (DE)
(72) Erfinder: Jonas, Rochus, Dr., D-64291 Darmstadt (DE); Lues, Inge, Dr., D-64297 Darmstadt (DE); Beier, Norbert, Dr., D-64354 Reinheim (DE); Minck, Klaus-Otto, Dr., D-64372 Ober-Ramstadt (DE)

(56) Entgegenhaltungen:
- EP-A- 0 294 647
- EP-A- 0 579 077
- EP-A- 0 618 201

## Beschreibung

Die Erfindung betrifft neue 3-Alkoxycarbonyl-thiadiazinone der Formel I 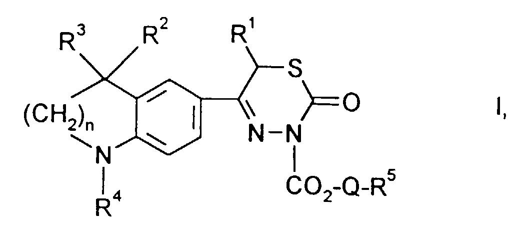 worin
- R¹, R² und R³: jeweils unabhängig voneinander H oder A,
- R⁴: Acyl mit 1 bis 15 C-Atomen,
- R⁵: NH₂, NHA, NA₂ oder einen gesättigten drei- bis achtgliedrigen heterocyclischen Rest mit mindestens einem N-Atom, welches durch A substituiert sein kann und worin zusätzlich eine weitere CH₂-Gruppe im Ring durch eine NH-, NA-Gruppe, ein O- oder ein S-Atom ersetzt sein kann,
- Q: fehlt oder verzweigtes oder unverzweigtes Alkylen mit 1 bis 10 C-Atomen,
- n: 1, 2 oder 3 und
- A: Alkyl mit 1 bis 6 C-Atomen
bedeuten,
sowie deren physiologisch unbedenkliche Salze.

Thiadiazinonderivate, deren Grundstruktur der Formel I entspricht, die jedoch ansonsten ein anderes Substitutionsmuster aufweisen, sind aus DE 37 19 031 A1 bekannt.

Thiadiazinonderivate mit anderem Substitutionsmuster zur Behandlung cardiovaskulärer Erkrankungen sind auch in der EP 579 077 und in der EP 618 201 beschrieben.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I bei guter Verträglichkeit wertvolle pharmakologische Eigenschaften besitzen. Insbesondere zeigen sie eine starke antiarrhythmische Wirkung sowie einen positiv inotropen Effekt; ferner wirken die Substanzen vasodilatierend und fördem daher die Durchblutung. Die vasodilatierende und die Herzwirkung kann z.B. an narkotisierten oder wachen Hunden, Katzen, Affen oder Minischweinen, die positiv inotrope Wirkung auch an isolierten Herzpräparationen (z.B. Vorhof, Papillarmuskel oder perfundiertes Ganzherz) von Ratte, Meerschweinchen, Katze oder Hund ermittelt werden, z.B. nach Methoden, wie sie in Arzneimittelforschung, Band 31 (I) Nr. 1a (1981), Seiten 141 bis 170, oder von Schliep et al. im 9th Intemational Congress of Pharmacol., London, Abstracts of papers 9P (1984), beschrieben sind.

Weiterhin treten antithrombotische, thrombozytenaggregationshemmende und die Erythrozytenform beeinflussende Eigenschaften auf. Die Beeinflussung der Thrombozytenfunktion im Sinne einer Aggregationshemmung kann an der Ratte ex vivo im Test nach Born (Nature 194, 927-929, 1962) nachgewiesen werden. Die antithrombotische Wirkung zeigt sich in der Verlängerung der Blutungszeit nach Stella (Thrombos. Res. 7, 709-716, 1975) in der Verminderung des Thrombusgewichtes bei der kälteinduzierten Thrombosierung der Jugular-Vene bei der Ratte nach Meng (Ther. Ber. 47, 69-79, 1975) und der Erhöhung der zur vollständigen Thrombosierung notwendigen Laserimpulse an der Mesenterial-Venole der Ratte, entsprechend einer Abwandlung der Methode nach Kovacs (Microvasc. Res. 6, 194-201, 1973).

Die günstige Wirkung auf die Erythrozytenverformbarkeit ist im Nucleoporefilter nach Schmid-Schönbein (Pflüger's Archiv 338, 93-114, 1973) nachweisbar. Auch lassen sich günstige Effekte auf die Fibrinolyse/ Euglobulinlysiszeit nach v. Kaulla (Progr. Chem. Fibrinol., Thrombol. 1, 131-149, 1975; ed J.F. Davidson, Raven Press, N.Y.) feststellen.

Ferner wurde gefunden, daß die Einführung basisch substituierter Alkoxycarbonylreste am Thiadiazinonring zu gut wasserlöslichen Verbindungen führt, die eine gute Bioverfügbarkeit aufweisen.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe dienen.

Gegenstand der Erfindung sind dementsprechend die Verbindungen der Formel I, ihre Säureadditionssalze sowie ein Verfahren zu ihrer Herstellung, dadurch gekennzeichnet, daß man eine Verbindung der Formel II 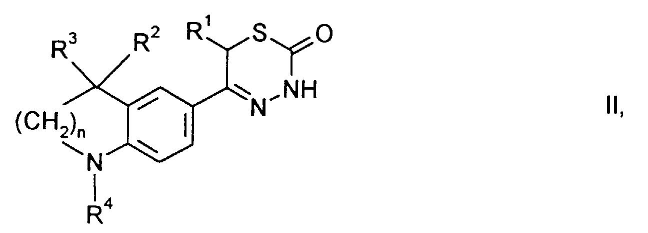 worin
R¹, R², R³, R⁴ und n die angegebenen Bedeutungen haben, mit einer Verbindung der Formel III

X-CO₂-Q-R⁵ III,

worin Q und R⁵ die angegebenen Bedeutungen haben und X Cl, Br, OH oder eine reaktionsfähige veresterte OH-Gruppe bedeutet,
umsetzt,
oder daß man eine Verbindung der Formel IV 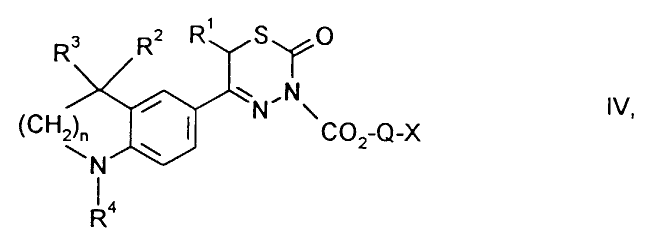 worin
R¹, R², R³, R⁴, n, Q und X die angegebenen Bedeutungen besitzen, mit einer Verbindung der Formel V

R⁵-H V,

worin
R⁵ die angegebene Bedeutung hat,
umsetzt,
oder daß man eine Verbindung, die der Formel I entspricht, jedoch anstelle von R⁴ ein Wasserstoffatom besitzt, auf übliche Weise acyliert,
oder daß man in einer Verbindung der Formel I einen Rest R⁴ in einen anderen Rest R⁴ umwandelt,
oder daß man eine Verbindung der Formel I, worin der Rest R⁵ eine primäre oder eine sekundäre Aminogruppe enthält, auf übliche Weise alkyliert,
und/oder daß man eine Base der Formel I durch Behandeln mit einer Säure in eines ihrer Salze umwandelt.

Vor- und nachstehend haben die Reste R¹, R², R³, R⁴, R⁵, Q und X sowie der Parameter n die bei den Formeln I, II, III, IV und V angegebenen Bedeutungen, wenn nicht ausdrücklich etwas anderes angegeben ist.

In den Formeln ist A vorzugsweise unverzweigt, hat vorzugsweise 1, 2, 3 oder 4 C-Atome und steht bevorzugt für Methyl, ferner bevorzugt für Ethyl oder Propyl, weiterhin bevorzugt für Isopropyl, Butyl, Isobutyl, sek.-Butyl, tert.-Butyl, aber auch für n-Pentyl, Isopentyl, n-Hexyl oder Isohexyl.

Q bedeutet vorzugsweise Ethylen, Propylen, Butylen, Pentylen, Hexylen, ferner z.B. auch 1-Methylethylen.

Q-R⁵ bedeutet vorzugsweise auch N-Methylpiperidyl.

Die Reste R¹, R² und R³ bedeuten vorzugsweise jeweils H oder Methyl.

n bedeutet vorzugsweise 2.

R⁴ bedeutet den Säurerest einer Carbon- oder Sulfonsäure, vorzugsweise Alkanoyl mit 1-10, insbesondere 1, 2, 3, 4 oder 5 C-Atomen, im einzelnen bevorzugt Acetyl, ferner bevorzugt Formyl, Propionyl, Butyryl, Isobutyryl, Valeryl, Isovaleryl, Pivaloyl (Trimethylacetyl), weiterhin bevorzugt gegebenenfalls substituiertes Aroyl mit 7-15 C-Atomen, wobei als Substituenten insbesondere 1-3, vorzugsweise eine der folgenden Gruppen in Frage kommen: Alkyl, Alkoxy, Alkylthio, Alkylsulfinyl oder Alkylsulfonyl mit jeweils 1-3, vorzugsweise 1 oder 2 C-Atomen, Methylendioxy, ferner OH, F, Cl, Br, J, NO₂, NH₂, Alkylamino oder Dialkylamino mit jeweils 1-3, vorzugsweise 1 oder 2 C-Atomen in der Alkylgruppe. Einzelne bevorzugte Aroylreste sind Benzoyl, o-, m- oder p-Toluyl, o-, m- oder p-Methoxybenzoyl, 2,3-, 2,4-, 2,5-, 2,6-, 3,4- oder 3,5-Dimethoxybenzoyl, 2,3,4-, 2,3,5-, 2,3,6-, 2,4,5-, 2,4,6- oder 3,4,5-Trimethoxybenzoyl, o-, m- oder p-Methylthiobenzoyl, o-, m- oder p-Methylsulfinylbenzoyl, o-, m- oder p-Methylsulfonylbenzoyl, 2,3- oder 3,4-Methylendioxybenzoyl, 1- oder 2-Naphthoyl Acyl kann weiterhin für Heteroarylcarbonyl mit 2-10 C-Atomen wie 2- oder 3-Furoyl, 2- oder 3-Thenoyl, Picolinoyl, Nicotinoyl, Isonicotinoyl stehen, außerdem für Arylalkanoyl wie Phenylacetyl, o-, m- oder p-Methoxyphenylacetyl, 2- oder 3-Phenylpropionyl, 2-, 3- oder 4-Phenylbutyryl; für Cycloalkylcarbonyl wie Cyclohexylcarbonyl; für Alkylsulfonyl wie Methyl-, Ethyl-, Propyl- oder Butylsulfonyl; für Arylsulfonyl wie Benzolsulfonyl, o-, m- oder p-Toluolsulfonyl, o-, m- oder p-Methoxybenzolsulfonyl, 1- oder 2-Naphthalinsulfonyl.

Der Rest R⁵ bedeutet vorzugsweise Methylamino, Dimethylamino, Ethylamino, Methylethylamino, Diethylamino oder aber Pyrrolidino, Piperidino oder Morpholino.

Gegenstand der Erfindung sind insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ih ausgedrückt werden, die der Formel I entsprechen und worin die nicht näher bezeichneten Reste die bei der Formel I angegebene Bedeutung haben, worin jedoch
in Ia
   - R¹: Methyl und
   - n: 2 bedeuten;
in Ib
   - R¹: H und
   - n: 2 bedeuten;
in Ic
   - R¹: Methyl,
   - R² und R³: H und
   - n: 2 bedeuten;
in Id
   - R¹ und R²: Methyl,
   - R³: H und
   - n: 2 bedeuten;
in Ie
   - R¹, R² und R³: Methyl und
   - n: 2 bedeuten;
in If
   - R¹, R² und R³: Methyl,
   - R⁴: Acyl mit 1-10 C-Atomen und
   - n: 2 bedeuten;
in Ig
   - R¹ und R²: jeweils unabhängig voneinander H oder Methyl,
   - R³: H,
   - R⁴: Acyl mit 1-10 C-Atomen,
   - Q: verzweigtes oder unverzweigtes Alkylen mit 1-6 C-Atomen,
   - R⁵: NH₂, NHCH₃, NHC₂H₅, N(CH₃)₂ oder N(C₂H₅)₂ und
   - n: 2 bedeuten;
in Ih
   - R¹, R² und R³: Methyl,
   - R⁴: Acyl mit 1-10 C-Atomen und
   - Q-R⁵: Pyrrolidinoethyl, Pyrrolidinopropyl, Piperidinoethyl, Piperidinopropyl, Morpholinoethyl, Morpholinopropyl, N-Methylpyrrolidinyl, N-Ethylpyrrolidinyl, N-Methylpiperidinyl, N-Ethylpiperidinyl oder Morpholinyl bedeuten.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondem sofort weiter zu den Verbindungen der Formel I umsetzt.

Die Ausgangsstoffe der Formeln II und III sind teilweise bekannt. Sofem sie nicht bekannt sind, können sie nach an sich bekannten Methoden hergestellt werden. Die Herstellung der Verbindungen der Formel II ist aus DE 37 19 031 bekannt.

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa +150°, vorzugsweise zwischen 20 und 100°. Als Lösungsmittel eignen sich beispielsweise Kohlenwasserstoffe wie Benzol, Toluol, Xylole oder Mesitylen; halogenierte Kohlenwasserstoffe wie Dichlormethan, Trichlorethylen oder Chlorbenzol; Alkohole wie Methanol, Ethanol oder Isopropanol; Glykole oder Glykolether wie Ethylenglykol, Diethylenglykol, 2-Methoxyethanol; Nitrile wie Acetonitril; Ether wie Tetrahydrofuran oder Dioxan; Amide wie Dimethylformamid (DMF); Sulfoxide wie Dimethylsulfoxid. Auch Gemische dieser Lösungsmittel sind geeignet.

In den Verbindungen der Formel III steht X bevorzugt für Cl oder Br. Falls X eine reaktionsfähige veresterte OH-Gruppe bedeutet, so ist diese vorzugsweise Alkylsulfonyloxy mit 1-6 C-Atomen, z.B. Methansulfonyloxy oder Arylsulfonyloxy mit 6-10 C-Atomen, z.B. Benzol-, p-Toluol- oder 1- oder 2-Naphthalinsulfonyloxy.

Weiterhin kann man eine Verbindung der Formel I auch herstellen, indem man eine Verbindung der Formel IV mit einer Verbindung der Formel V umsetzt.

Verbindungen der Formel IV kann man dadurch erhalten, daß man eine Verbindung der Formel II mit einer Verbindung der Formel Y-CO₂-Q-OH, worin Y Cl, Br oder eine reaktionsfähige veresterte OH-Gruppe bedeutet, unter Bedingungen, wie sie für die Reaktion zwischen Verbindungen der Formeln II und III zuvor angegeben wurden, umsetzt, und daß man anschließend gewünschtenfalls die endständige OH-Gruppe funktionalisiert.

Verbindungen der Formel V sind bekannt oder können nach an sich bekannten Methoden hergestellt werden.

Ebenso ist es möglich, einen Rest R⁴ in einen anderen Rest R⁴ umzuwandeln. Man kann z.B., unter Verwendung von an sich bekannten Reaktionen, eine OH-Gruppe verethern oder aber einen Arylether spalten. Desweiteren können Substituenten des Restes R⁴, wie z.B. S-A- oder SO-A-Gruppen oxidiert werden, sofern die Reaktion selektiv am Rest R4 stattfindet.

Eine Verbindung, die der Formel I entspricht, aber anstelle von R⁴ ein Wasserstoffatom besitzt, kann mit einem Acylhalogenid der Formel R⁴⁻Cl oder R⁴-Br oder einem Anhydrid der Formel (R⁴)₂O in einem inerten Lösungsmittel acyliert werden, zweckmäßig in Gegenwart einer Base, z.B. eines Alkalimetalls- oder Erdalkalimetallhydroxids, -carbonats, -alkoholats oder -hydrids wie Natrium- oder Kaliumhydroxid, -carbonat, -methylat,-ethylat oder -hydrid, ferner sekundäre oder tertiäre Amine, z.B. Triethylamin oder Pyridin.

Eine erhaltene Base der Formel I kann mit einer Säure in das zugehörige Säureadditionssalz überführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- und Ethansulfonsäure, Ethandisulfonsäure, 2-Hydroxyethansulfonsäure, Benzolsulfonsäure, p-Toluolsulfonsäure, Naphthalinmono- und disulfonsäuren, Laurylschwefelsäure. Salze mit physiologisch nicht unbedenklichen Säuren, z.B. Pikrate, können zur Aufreinigung der Verbindungen der Formel I verwendet werden.

Verbindungen der Formel I können ein oder mehrere Asymmetriezentren enthalten. In diesem Fall liegen sie gewöhnlich in racemischer Form vor. Erhaltene Racemate können nach an sich bekannten Methoden mechanisch oder chemisch in ihre Enantiomeren getrennt werden. Vorzugsweise werden aus dem racemischen Gemisch durch Umsetzung mit einem optisch-aktiven Trennmittel Diastereomere gebildet. Als Trennmittel für basische Verbindungen der Formel I eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch-aktiven Camphersulfonsäuren, wie β-Camphersulfonsäure oder aber optisch aktive Camphansäure bzw. andere optisch aktive Terpensäuren.

Natürlich ist es auch möglich, optisch aktive Verbindungen der Formel I nach den oben beschriebenen Methoden zu erhalten, indem man Ausgangsstoffe verwendet, die bereits optisch aktiv sind.

Die Formel I umschließt alle Stereoisomeren und deren Gemische, z.B. die Racemate.

Gegenstand der Erfindung ist ferner die Verwendung der Verbindungen der Formel I und ihrer physiologischen unbedenklichen Salze zur Herstellung pharmazeutischer Zubereitungen, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, femer Suspensionen, Emulsionen oder Implantate, für die topische Anwendung Salben, Cremes, Sticks oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I können bei der Bekämpfung von Krankheiten, insbesondere von Arrhythmien und von Herzinsuffizienzen sowie bei der therapeutischen Behandlung des menschlichen oder tierischen Körpers verwendet werden.

Dabei werden die erfindungsgemäßen Substanzen in der Regel in Analogie zu bekannten positiv inotrop wirksamen Substanzen wie Amrinon verabreicht, vorzugsweise in Dosierungen zwischen etwa 1 und 100 mg, insbesondere zwischen 2 und 20 mg pro Dosierungseinheit.

Die tägliche Dosierung liegt vorzugsweise zwischen etwa 0,2 und 20 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

In den nachfolgenden Beispielen bedeutet "übliche Aufarbeitung" :

Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatographie und/oder Kristallisation. Rf-Werte an Kieselgel.

Vor- und nachstehend sind alle Temperaturen in Celsiusgraden angegeben.

### Beispiel 1

Zu einer Lösung von 10 g 3,6-Dihydro-5-[1,2,3,4-tetrahydro-1-(3,4-dimethoxybenzoyl)-6-chinolyl]-6-methyl-2H-1,3,4-thiadiazin-2-on ("A") und 9,8 ml Triethylamin in 100 ml Dichlormethan werden 6 g Chlorameisensäure-2-N,N-diethylaminoethylester, gelöst in 50 ml Dichlormethan, unter Rühren zugetropft und eine Stunde bei 20° nachgerührt. Man entfernt das Lösungsmittel, arbeitet wie üblich auf und erhält 3-[2-(N,N-Diethylamino)ethoxycarbonyl]-5-[1,2,3,4-tetrahydro-1-(3,4-dimethoxybenzoyl)-6-chinolyl]-6-methyl-3,6-dihydro-2H-1,3,4-thiadiazin-2-on; Hydrochlorid.

Analog erhält man durch Umsetzung von "A"
mit Chlorameisensäure-3-N,N-diethylamino-propylester:
   3-[3-(N, N-Diethylamino)-propoxycarbonyl]-5-[1,2,3,4-tetrahydro-1-(3, 4-dimethoxybenzoyl)-6-chinolyl]-6-methyl-3,6-dihydro-2H-1,3,4-thiadiazin-2-on; Hydrochlorid;
mit Chlorameisensäure-6-N,N-dimethylamino-hexylester:
   3-[6-(N, N-Dimethylamino)-hexyloxycarbonyl]-5-[1,2,3,4-tetrahydro-1-(3,4-dimethoxybenzoyl)-6-chinolyl]-6-methyl-3,6-dihydro-2H-1,3,4-thiadiazin-2-on; Hydrochlorid;
mit Chlorameisensäure-2-N,N-dimethylamino-1-methyl-ethylester:
   3-[1-Methyl-2-(N,N-dimethylamino)-ethoxycarbonyl]-5-[1,2,3,4-tetrahydro-1-(3,4-dimethoxybenzoyl)-6-chinolyl]-6-methyl-3,6-dihydro-2H-1,3,4-thiadiazin-2-on; Hydrochlorid;
mit Chlorameisensäure-(1-Methyl-4-piperidyl)-ester:
   3-(1-Methyl-4-piperidyloxycarbonyl)-5-[1,2,3,4-tetrahydro-1-(3,4-dimethoxybenzoyl)-6-chinolyl]-6-methyl-3,6-dihydro-2H-1,3,4-thiadiazin-2-on; Hemifumarat;
mit Chlorameisensäure-2-morpholino-ethylester:
   3-(2-Morpholino-ethoxycarbonyl)-5-[1,2,3,4-tetrahydro-1-(3,4-dimethoxybenzoyl)-6-chinolyl]-6-methyl-3,6-dihydro-2H-1,3,4-thiadiazin-2-on;
mit Chlorameisensäure-3-morpholino-propylester:
   3-(3-Morpholino-propoxycarbonyl)-5-[1,2,3,4-tetrahydro-1-(3,4-dimethoxybenzoyl)-6-chinolyl]-6-methyl-3,6-dihydro-2H-1,3,4-thiadiazin-2-on;
mit Chlorameisensäure-2-thiomorpholino-ethylester:
   3-(2-Thiomorpholino-ethoxycarbonyl)-5-[1,2,3,4-tetrahydro-1-(3,4-dimethoxybenzoyl)-6-chinolyl]-6-methyl-3,6=dihydro-2H-1,3,4-thiadiazin-2-on;
mit Chlorameisensäure-3-thiomorpholino-propylester:
   3-(3-Thiomorpholino-propoxycarbonyl)-5-[1,2,3,4-tetrahydro-1-(3,4-dimethoxybenzoyl)-6-chinolyl]-6-methyl-3,6-dihydro-2H-1,3,4-thiadiazin-2-on;
mit Chlorameisensäure-2-piperazino-ethylester:
   3-(2-Piperazino-ethoxycarbonyl)-5-[1,2,3,4-tetrahydro-1-(3,4-dimethoxybenzoyl)-6-chinolyl]-6-methyl-3,6-dihydro-2H-1,3,4-thiadiazin-2-on;
mit Chlorameisensäure-3-piperazino-propylester:
   3-(3-Piperazino-propoxycarbonyl)-5-[1,2,3,4-tetrahydro-1-(3,4-dimethoxybenzoyl)-6-chinolyl]-6-methyl-3,6-dihydro-2H-1,3,4-thiadiazin-2-on;
mit Chlorameisensäure-2-pyrrolidino-ethylester:
   3-(2-Pyrrolidino-ethoxycarbonyl)-5-[1 ,2, 3,4-tetrahydro-1-(3,4-dimethoxybenzoyl)-6-chinolyl]-6-methyl-3,6-dihydro-2H-1,3,4-thiadiazin-2-on;
mit Chlorameisensäure-3-pyrrolidino-propylester:
   3-(3-Pyrrolidino-propoxycarbonyl)-5-[1,2,3,4-tetrahydro-1-(3,4-dimethoxybenzoyl)-6-chinolyl]-6-methyl-3,6-dihydro-2H-1,3,4-thiadiazin-2-on.

Analog erhält man durch Umsetzung von (+)-3,6-Dihydro-5-[1,2,3,4-tetrahydro-1-(3,4-dimethoxybenzoyl)-6-chinolyl]-6-methyl-2H-1,3,4-thiadiazin-2-on
mit Chlorameisensäure-2-N,N-diethylamino-ethylester:
   (+)-3-[2-(N,N-Diethylamino)-ethoxycarbonyl]-5-[1,2,3,4-tetrahydro-1-(3,4-dimethoxybenzoyl)-6-chinolyl]-6-methyl-3,6-dihydro-2H-1,3,4-thiadiazin-2-on; Hydrochlorid, F. 135;
mit Chlorameisensäure-3-N,N-diethylamino-propylester:
   (+)-3-[3-(N,N-Diethylamino)-propoxycarbonyl]-5-[1,2,3,4-tetrahydro-1-(3,4-dimethoxybenzoyl)-6-chinolyl]-6-methyl-3,6-dihydro-2H-1,3,4-thiadiazin-2-on; Hydrochlorid, F. 130;
mit Chlorameisensäure-6-N,N-diethylamino-hexylester:
   (+)-3-[6-(N,N-Dimethylamino)-hexyloxycarbonyl]-5-[1,2,3,4-tetrahydro-1-(3,4-dimethoxybenzoyl)-6-chinolyl]-6-methyl-3,6-dihydro-2H-1,3,4-thiadiazin-2-on; Hydrochlorid, F. 133;
mit Chlorameisensäure-6-N,N-diethylamino-1-methyl-ethylester:
   (+)-3-[1-Methyl-2-(N,N-dimethylamino)-ethoxycarbonyl]-5-[1,2,3,4-tetrahydro-1-(3,4,dimethoxybenzoyl)-6-chinolyl]-6-methyl-3,6-dihydro-2H-1,3,4-thiadiazin-2-on; Hydrochlorid, F. 168;
mit Chlorameisensäure-(1-Methyl-4-piperidyl)-ester:
   (+)-3-[1-Methyl-4-piperidyloxycarbonyl)-5-[1,2,3,4-tetrahydro-1-(3,4-dimethoxybenzoyl)-6-chinolyl]-6-methyl-3,6-dihydro-2H-1,3,4-thiadiazin-2-on; Hydrochlorid, F. 150;

Analog erhält man durch Umsetzung von Chlorameisensäure-2-N,N-diethylaminoethylester
mit 5-[1,2,3,4-Tetrahydro-1-(3-ethoxy-4-methoxybenzoyl)-6-chinolyl]-6-methyl-3,6-dihydro-2H-1,3,4-thiadiazin-2-on:
   3-[2-(N,N-Diethylamino)-ethoxycarbonyl]-5-[1,2,3,4-tetrahydro-1-(3-ethoxy-4-methoxybenzoyl)-6-chinolyl]-6-methyl-3,6-dihydro-2H-1,3,4-thiadiazin-2-on;
mit 5-[1,2,3,4-Tetrahydro-1-(3-methoxy-4-ethoxybenzoyl)-6-chinolyl]-6-methyl-3,6-dihydro-2H-1,3,4-thiadiazin-2-on:
   3-[2-(N,N-Diethylamino)-ethoxycarbonyl]-5-[1,2,3,4-tetrahydro-1-(3-methoxy-4-ethoxybenzoyl)-4-chinolyl]-6-methyl-3,6-dihydro-2H-1,3,4-thiadiazin-2-on;
mit 5-[1,2,3,4-Tetrahydro-1-(3-methoxy-4-hydroxybenzoyl)-6-chinolyl]-6-methyl-3,6-dihydro-2H-1,3,4-thiadiazin-2-on:
   3-[2-(N,N-Diethylamino)-ethoxycarbonyl]-5-[1,2,3,4-tetrahydro-1-(3-methoxy-4-hydroxybenzoyl)-6-chinolyl]-6-methyl-3,6-dihydro-2H-1,3,4-thiadiazin-2-on.

### Beispiel 2

Zu einer Lösung von 10 g 3,6-Dihydro-5-[1,2,3,4-tetrahydro-1-(3,4-dimethoxybenzoyl)-4,4-dimethyl-6-chinolyl]-6-methyl-2H-1,3,4-thiadiazin-2-on ("B") und 9,8 ml Pyridin in 100 ml Dichlormethan werden 6 g Chlorameisensäure-2-N,N-diethylamino-ethylester, gelöst in 50 ml Dichlormethan, unter Rühren zugetropft und eine Stunde bei 20° nachgerührt. Man entfernt das Lösungsmittel, arbeitet wie üblich auf und erhält 3-[2-(N,N-Diethylamino)-ethoxycarbonyl]-5-[1,2,3,4-tetrahydro-1-(3,4-dimethoxybenzoyl)-4,4-dimethyl-6-chinolyl]-6-methyl-3,6-dihydro-2H-1,3,4-thiadiazin-2-on; Rf (THF) 0,65.

Analog erhält man durch Umsetzung von "B"
mit Chlorameisensäure-3-N,N-diethylamino-propylester:
   3-[3-(N,N-Diethylamino)-propoxycarbonyl]-5-[1,2,3,4-tetrahydro-1-(3,4-dimethoxybenzoyl)-4,4-dimethyl-6-chinolyl]-6-methyl-3,6-dihydro-2H-1,3,4-thiadiazin-2-on; Rf (THF) 0,64;
mit Chlorameisensäure-6-N,N-dimethylamino-hexylester:
   3-[6-(N,N-Dimethylamino)-hexyloxycarbonyl]-5-[1,2,3,4-tetrahydro-1-(3,4-dimethoxybenzoyl)-4,4-dimethyl-6-chinolyl]-6-methyl-3,6-dihydro-2H-1,3,4-thiadiazin-2-on; Rf (THF) 0,2;
mit Chlorameisensäure-2-N,N-dimethylamino-1-methyl-ethylester:
   3-[1-Methyl-2-(N,N-dimethylamino)-ethoxycarbonyl]-5-[1,2,3,4-tetrahydro-1-(3,4-dimethoxybenzoyl)-4,4-dimethyl-6-chinolyl]-6-methyl-3,6-dihydro-2H-1,3,4-thiadiazin-2-on; Rf (THF) 0,25;
mit Chlorameisensäure-(1-Methyl-4-piperidyl)-ester:
   3-(1-Methyl-4-piperidyloxycarbonyl)-5-[1,2,3,4-tetrahydro-1-(3,4-dimethoxybenzoyl)-4,4-dimethyl-6-chinolyl]-6-methyl-3,6-dihydro-2H-1,3,4-thiadiazin-2-on; Hemifumarat; Rf (THF) 0,65.

Analog erhält man durch Umsetzung von Chlorameisensäure-2-N,N-diethylaminoethylester
mit 5-[1,2,3,4-Tetrahydro-1-(3-ethoxy-4-methoxybenzoyl)-6-chinolyl]-4,4-dimethyl-6-methyl-3,6-dihydro-2H-1,3,4-thiadiazin-2-on:
   3-[2-(N,N-Diethylamino)-ethoxycarbonyl]-5-[1,2,3,4-tetrahydro-1-(3-ethoxy-4-methoxybenzoyl)-4,4-dimethyl-6-chinolyl]-6-methyl-3,6-dihydro-2H-1,3,4-thiadiazin-2-on;
mit 5-[1,2,3,4-Tetrahydro-1-(3-methoxy-4-ethoxybenzoyl)-6-chinolyl]-4,4-dimethyl-6-methyl-3,6-dihydro-2H-1,3,4-thiadiazin-2-on:
   3-[2-(N, N-Diethylamino)-ethoxycarbonyl]-5-[1,2,3,4-tetrahydro-1-(3-methoxy-4-ethoxybenzoyl)-4,4-dimethyl-6-chinolyl]-6-methyl-3,6-dihydro-2H-1,3,4-thiadiazin-2-on;
mit 5-[1,2,3,4-Tetrahydro-1-(3-methoxy-4-hydroxybenzoyl)-4,4-dimethyl-6-chinolyl]-6-methyl-3,6-dihydro-2H-1,3,4-thiadiazin-2-on:
   3-[2-(N,N-Diethylamino)-ethoxycarbonyl]-5-[1,2,3,4-tetrahydro-1-(3-methoxy-4-hydroxybenzoyl)-4,4-dimethyl-6-chinolyl]-6-methyl-3,6-dihydro-2H-1,3,4-thiadiazin-2-on.

### Beispiel 3

Zu einer Lösung von 10 g 3-[2-Chlorethoxycarbonyl]-5-[1,2,3,4-tetrahydro-1-(3,4-dimethoxybenzoyl)-6-chinolyl]-6-methyl-3,6-dihydro-2H-1,3,4-thiadiazin-2-on und 9 ml Pyridin in 100 ml Dichlormethan werden 2,5 g Diethylamin, gelöst in 30 ml Dichlormethan, unter Rühren zugetropft und eine Stunde bei 20° nachgerührt. Man entfernt das Lösungsmittel, arbeitet wie üblich auf und erhält 3-[2-(N,N-Diethylamino)-ethoxycarbonyl]-5-[1,2,3,4-tetrahydro-1-(3,4-dimethoxybenzoyl)-6-chinolyl]-6-methyl-3,6-dihydro-2H-1,3,4-thiadiazin-2-on; Hydrochlorid.

### Beispiel 4

Analog Beispiel 1 erhält man durch Umsetzung von Chlorameisensäure-3-N, N-diethylaminopropylester
mit 5-[1,2,3,4-Tetrahydro-1-(3-ethoxy-4-methoxybenzoyl)-6-chinolyl]-6-methyl-3,6-dihydro-2H-1,3,4-thiadiazin-2-on:
   3-[3-(N,N-Diethylamino)-propoxycarbonyl]-5-[1,2,3,4-tetrahydro-1-(3-ethoxy-4-methoxybenzoyl)-6-chinolyl]-6-methyl-3,6-dihydro-2H-1,3,4-thiadiazin-2-on;
mit 5-[1,2,3,4-Tetrahydro-1-(3-methoxy-4-ethoxybenzoyl)-6-chinolyl]-6-methyl-3,6-dihydro-2H-1,3,4-thiadiazin-2-on:
   3-[3-(N,N-Diethylamino)-propoxycarbonyl]-5-[1,2,3,4-tetrahydro-1-(3-methoxy-4-ethoxybenzoyl)-6-chinolyl]-6-methyl-3,6-dihydro-2H-1,3,4-thiadiazin-2-on;
mit 5-[1,2,3,4-Tetrahydro-1-(3-methoxy-4-hydroxybenzoyl)-6-chinolyl]-6-methyl-3,6-dihydro-2H-1,3,4-thiadiazin-2-on:
   3-[3-(N,N-Diethylamino)-propoxycarbonyl]-5-[1,2,3,4-tetrahydro-1-(3-methoxy-4-hydroxybenzoyl)-6-chinolyl]-6-methyl-3,6-dihydro-2H-1,3,4-thiadiazin-2-on.

### Beispiel 5

Analog Beispiel 2 erhält man durch Umsetzung von Chlorameisensäure-3-N,N-diethylaminopropylester
mit 5-[1,2,3,4-Tetrahydro-1-(3-ethoxy-4-methoxybenzoyl)-4,4-dimethyl-6-chinolyl]-6-methyl-3,6-dihydro-2H-1,3,4-thiadiazin-2-on:
   3-[3-(N,N-Diethylamino)-propoxycarbonyl]-5-[1,2,3,4-tetrahydro-1-(3-ethoxy-4-methoxybenzoyl)-4,4-dimethyl-6-chinolyl]-6-methyl-3,6-dihydro-2H-1,3,4-thiadiazin-2-on;
mit 5-[1,2,3,4-Tetrahydro-1-(3-methoxy-4-ethoxybenzoyl)-4,4-dimethyl-6-chinolyl]-6-methyl-3,6-dihydro-2H-1,3,4-thiadiazin-2-on:
   3-[3-(N,N-Diethylamino)-propoxycarbonyl]-5-[1,2,3,4-tetrahydro-1-(3-methoxy-4-ethoxybenzoyl)-4,4-dimethyl-6-chinolyl]-6-methyl-3,6-dihydro-2H-1,3,4-thiadiazin-2-on;
mit 5-[1,2,3,4-Tetrahydro-1-(3-methoxy-4-hydroxybenzoyl)-4,4-dimethyl-6-chinolyl]-6-methyl-3,6-dihydro-2H-1,3,4-thiadiazin-2-on:
   3-[3-(N,N-Diethylamino)-propoxycarbonyl]-5-[1,2,3,4-tetrahydro-1-(3-methoxy-4-hydroxybenzoyl)-4,4-dimethyl-6-chinolyl]-6-methyl-3,6-dihydro-2H-1,3,4-thiadiazin-2-on.

### Beispiel 6

Eine Lösung von 12 g 3-[2-(N,N-Diethylamino)-ethoxycarbonyl]-5-(1,2,3,4-tetrahydro-6-chinolyl)-6-methyl-3,-dihydro-2H-1,3,4-thiadiazin-2-on [erhältlich analog Beispiel 1 durch Reaktion von 5-(1,2,3,4-Tetrahydro-6-chinolyl)-6-methyl-3,6-dihydro-2H-1,3,4-thiadiazin-2-on mit Chlorameisensäure-2-N,N-diethylaminoethylester] in 100 ml Dichlormethan wird mit 5 ml Triethylamin, dann tropfenweise unter Rühren mit 4 ml Acetylchlorid versetzt. Man rührt noch eine Stunde bei 20°, zersetzt mit Wasser, arbeitet wie üblich auf und erhält 3-[2-(N,N-Diethylamino)-ethoxycarbonyl]-5-(1-acetyl-1,2,3,4-tetrahydro-6-chinolyl)-6-methyl-3,6-dihydro-2H-1,3,4-thiadiazin-2-on.

Analog erhält man die nachstehenden 3-[2-(N,N-Diethylamino)-ethoxycarbonyl]-6-methyl-3,6-dihydro-2H-1,3,4-thiadiazin-2-one:
5-(1-Formyl-1,2,3,4-tetrahydro-6-chinolyl)-
5-(1-Propionyl-1,2,3,4-tetrahydro-6-chinolyl)-
5-(1-Butyryl-1,2,3,4-tetrahydro-6-chinolyl)-
5-(1-Isobutyryl-1,2,3,4-tetrahydro-6-chinolyl)-
5-(1-Valeryl-1,2,3,4-tetrahydro-4-chinolyl)-
5-(1-Isovaleryl-1,2,3,4-tetrahydro-6-chinolyl)-
5-(1-Pivaloyl-1,2,3,4-tetrahydro-6-chinolyl)-
5-(1-Benzoyl-1,2,3,4-tetrahydro-6-chinolyl)-
5-(1-p-Methoxy-benzoyl-1,2,3,4-tetrahydro-6-chinolyl)-
5-[1-(3,4-Methylendioxy-benzoyl)-1,2,3,4-tetrahydro-6-chinolyl]-
5-(1-p-Methylthio-benzoyl-1,2,3,4-tetrahydro-6-chinolyl)-
5-(1-p-Methylsulfinyl-benzoyl-1,2,3,4-tetrahydro-6-chinolyl)-
5-(1-p-Methylsulfonyl-benzoyl-1,2,3,4-tetrahydro-6-chinolyl)-
5-(1-Picolinoyl-1,2,3,4-tetrahydro-6-chinolyl)-
5-(1-Nicotinoyl-1,2,3,4-tetrahydro-6-chinolyl)-
5-(1-Isonicotinoyl-1,2,3,4-tetrahydro-6-chinolyl)-
5-(1-Methansulfonyl-1,2,3,4-tetrahydro-6-chinolyl)-
5-(1-Benzolsulfonyl-1,2,3,4-tetrahydro-6-chinolyl)-
5-(1-p-Toluolsulfonyl-1,2,3,4-tetrahydro-6-chinolyl)-
5-(1-Formyl-4,4-dimethyl-1,2,3,4-tetrahydro-6-chinolyl)-
5-(1-Acetyl-4,4-dimethyl-1,2,3,4-tetrahydro-6-chinolyl)-
5-(1-Propionyl-4,4-dimethyl-1,2,3,4-tetrahydro-6-chinolyl)-
5-(1-Butyryl-4,4-dimethyl-1,2,3,4-tetrahydro-6-chinolyl)-
5-(1-Isobutyryl-4,4-dimethyl-1,2,3,4-tetrahydro-6-chinolyl)-
5-(1-Valeryl-4,4-dimethyl-1,2,3,4-tetrahydro-6-chinolyl)-
5-(1-Isovaleryl-4,4-dimethyl-1,2,3,4-tetrahydro-6-chinolyl)-
5-(1-Pivaloyl-4,4-dimethyl-1,2,3,4-tetrahydro-6-chinolyl)-
5-(1-Benzoyl-4,4-dimethyl-1,2,3,4-tetrahydro-6-chinolyl)-
5-(1-p-Methoxy-benzoyl-4,4-dimethyl-1,2,3,4-tetrphydro-6-chinolyl)-
5-[1-(3,4-Methylendioxy-benzoyl)-4,4-dimethyl-1,2,3,4-tetrahydro-6-chinolyl]-
5-(1-p-Methylthio-benzoyl-4,4-dimethyl-1,2,3,4-tetrahydro-6-chinolyl)-
5-(1-p-Methylsulfinyl-benzoyl-4,4-dimethyl-1,2,3,4-tetrahydro-6-chinolyl)-
5-(1-p-Methylsulfonyl-benzoyl-4,4-dimethyl-1,2,3,4-tetrahydro-6-chinolyl)-
5-(1-Picolinoyl-4,4-dimethyl-1,2,3,4-tetrahydro-6-chinolyl)-
5-(1-Nicotinoyl-4,4-dimethyl-1,2,3,4-tetrahydro-6-chinolyl)-
5-(1-Isonicotinoyl-4,4-dimethyl-1,2,3,4-tetrahydro-6-chinolyl)-
5-(1-Methansulfonyl-4,4-dimethyl-1,2,3,4-tetrahydro-6-chinolyl)-
5-(1-Benzolsulfonyl-4,4-dimethyl-1,2,3,4-tetrahydro-6-chinolyl)-
5-(1-p-Toluolsulfonyl-4,4-dimethyl-1,2,3,4-tetrahydro-6-chinolyl)-.

### Beispiel 7

Analog Beispiel 1 erhält man durch Umsetzung von
3,6-Dihydro-5-[1,2,3,4-tetrahydro-1-(3-methoxy-4-ethoxybenzoyl)-6-chinolyl]-6-methyl-2H-1,3,4-thiadiazin-2-on mit Chlorameisensäure-3-morpholino-propylester:
   3-(3-Morpholino-propoxycarbonyl)-5-[1,2,3,4-tetrahydro-1-(3-methoxy-4-ethoxybenzoyl)-6-chinolyl]-6-methyl]-2H-1,3,4-thiadiazin-2-on;
von 3,6-Dihydro-5-[1,2,3,4-tetrahydro-1-(3-methoxy-4-ethoxybenzoyl)-6-chinolyl]-6-methyl-2H-1,3,4-thiadiazin-2-on mit Chlorameisensäure-3-piperidino-propylester:
   3-(3-Piperidino-propoxycarbonyl)-5-[1,2,3,4-tetrahydro-1-(3-methoxy-4-ethoxybenzoyl)-6-chinolyl]-6-methyl-2H-1,3,4-thiadiazin-2-on.

### Beispiel 8

Zu einer Lösung von 10 g 3-(3-Piperazino-propoxycarbonyl)-5-[1,2,3,4-tetrahydro-1-(3,4-dimethoxybenzoyl)-6-chinolyl]-6-methyl-3,6-dihydro-2H-1,3,4-thiadiazin-2-on und 1,8 g Triethylamin in 100 ml Toluol werden 1,3 g Ethylchlorid, gelöst in 10 ml Toluol, unter Rühren zugetropft, und eine Stunde unter Erwärmen gerührt. Man entfernt das Lösungsmittel, arbeitet wie üblich auf und erhält 3-[3-(1-Ethyl-4-piperazinyl)-propoxycarbonyl]-5-[1,2,3,4-tetrahydro-1-(3,4-dimethoxy-benzoyl)-6-chinolyl]-6-methyl-3,6-dihydro-2H-1,3,4-thiadiazin-2-on.

### Beispiel 9

Analog Beispiel 1 erhält man durch Umsetzung
von 3,6-Dihydro-5-[1,2,3,4-tetrahydro-1-(3-methoxy-4-ethoxybenzoyl)-4,4-dimethyl-6-chinolyl]-6-methyl-2H-1,3,4-thiadiazin-2-on mit Chlorameisensäure-3-morpholino-propylester:
   3-(3-Morpholino-propoxycarbonyl)-5-[1,2,3,4-tetrahydro-1-(3-methoxy-4-ethoxybenzoyl)-4,4-dimethyl-6-chinolyl]-6-methyl-3,6-dihydro-2H-1,3,4-thiadiazin-2-on;
von 3,6-Dihydro-5-[1,2,3,4-tetrahydro-1-(3-methoxy-4-ethoxybenzoyl)-4,4-dimethyl-6-chinolyl]-6-methyl-2H-1,3,4-thiadiazin-2-on mit Chlorameisensäure-3-piperidino-propylester:
   3-(3-Piperidino-propoxycarbonyl)-5-[1,2,3,4-tetrahydro-1-(3-methoxy-4-ethoxybenzoyl)-4,4-dimethyl-6-chinolyl]-6-methyl-3,6-dihydro-2H-1,3,4-thiadiazin-2-on;
von 3,6-Dihydro-5-[1,2,3,4-tetrahydro-1-(3,4-dimethoxybenzoyl)-6-chinolyl]-6-methyl-2H-1,3,4-thiadiazin-2-on mit Cl-C(=O)-O-C₁₀H₂₀-N(CH₃)₂:
   3-[10-(N, N-Dimethylamino)-decyloxycarbonyl]-5-[1,2,3,4-tetrahydro-1-(3,4-dimethoxybenzoyl)-6-chinolyl]-6-methyl-3,6-dihydro-2H-1,3,4thiadiazin-2-on, F. 87°;
von 3,6-Dihydro-5-[1,2,3,4-tetrahydro-1,-(3,4-dimethoxybenzoyl)-6-chinolyl]-6-methyl-2H-1,3,4-thiadiazin-2-on mit Chlorameisensäure-6-pyrrolidinohexylester:
   3-(6-Pyrrolidino-hexyloxycarbonyl)-5-[1,2,3,4-tetrahydro-1-(3,4-dimethoxybenzoyl)-6-chinolyl]-6-methyl-3,6-dihydro-2H-1,3,4-thiadiazin-2-on, F. 107°;

Die nachstehenden Beispiele betreffen pharmazeutische Zubereitungen, die Wirkstoffe der Formel I oder ihre Salze enthalten.

### Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

### Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

### Beispiel C: Weichgelatine-Kapseln

Übliche Weichgelatine-Kapseln werden mit einem Gemisch aus jeweils 50 mg Wirkstoff und 250 mg Olivenöl gefüllt.

### Beispiel D: Ampullen

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

### Beispiel E: Wässerige Suspension für orale Applikation

Eine wässerige Suspension des Wirkstoffs wird in üblicher Weise hergestellt. Die Einheitsdosis (5 ml) enthält 100 mg Wirkstoff, 100 mg Na-Carboxymethylcellulose, 5 mg Na-Benzoat und 100 mg Sorbit.

### Beispiel F: Suppositorien

Man schmilzt ein Gemisch von 20 g Wirkstoff mit 100 g Sojalecithin und 1400 g Kakaobutter, gießt in Formen und läßt erkalten. Jedes Suppositorium enthält 20 mg Wirkstoff.

## Patentansprüche

1. 3-Alkoxycarbonyl-thiadiazinone der Formel I 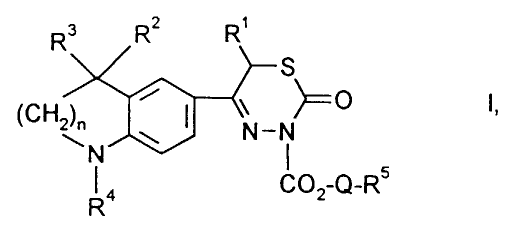 worin
R¹, R² und R³ jeweils unabhängig voneinander H oder A,
R⁴ Acyl mit 1 bis 15 C-Atomen,
R⁵ NH₂, NHA, NA₂ oder einen gesättigten drei- bis achtgliedrigen heterocyclischen Rest mit mindestens einem N-Atom, welches durch A substituiert sein kann und worin zusätzlich eine weitere CH₂-Gruppe im Ring durch eine NH-, NA-Gruppe, ein O- oder ein S-Atom ersetzt sein kann,
Q fehlt oder verzweigtes oder unverzweigtes Alkylen mit 1 bis 10 C-Atomen,
n 1, 2 oder 3 und
A Alkyl mit 1 bis 6 C-Atomen
bedeuten,
sowie deren physiologisch unbedenkliche Salze.

2. Ein Enantiomer einer Verbindung der Formel I gemäß Anspruch 1.

3. (a) 3-[2-(N,N-Diethylamino)-ethoxycarbonyl]-5-[1,2,3,4-tetrahydro-1-(3,4-dimethoxy-benzoyl)-6-chinolyl]-6-methyl-2H-3,6-dihydro1,3,4-thiadiazin-2-on;
(b) 3-[3-(N,N-Diethylamino)-propoxycarbonyl]-5-[1,2,3,4-tetrahydro-1-(3,4-dimethoxy-benzoyl)-6-chinolyl]-6-methyl-2H-3,6-dihydro-1,3,4-thiadiazin-2-on;
(c) 3-[6-(N,N-Dimethylamino)-hexyloxycarbonyl]-5-[1,2,3,4-tetrahydro-1-(3,4-dimethoxy-benzoyl)-6-chinolyl]-6-methyl-2H-3,6-dihydro-1,3,4-thiadiazin-2-on;
(d) 3-[1-Methyl-2-(N,N-dimethylamino)-ethoxycarbonyl]-5-[1,2,3,4-tetrahydro-1-(3,4-dimethoxy-benzoyl)-6-chinolyl]-6-methyl-2H3,6-dihydro-1,3,4-thiadiazin-2-on;
(e) 3-[1-Methyl-4-piperidyloxycarbonyl]-5-[1,2,3,4-tetrahydro-1-(3,4-dimethoxy-benzoyl)-6-chinolyl]-6-methyl-2H-3,6-dihydro-1,3,4-thiadiazin-2-on;
(f) 3-[2-(N,N-Diethylamino)-ethoxycarbonyl]-5-[1,2,3,4-tetrahydro-1-(3,4-dimethoxy-benzoyl)-4,4-dimethyl-6-chinolyl]-6-methyl-2H-3,6-dihydro-1,3,4-thiadiazin-2-on;
(g) 3-[6-(N,N-Dimethylamino)-hexyloxycarbonyl]-5-[1,2,3,4-tetrahydro-1-(3,4-dimethoxy-benzoyl)-4,4-dimethyl-6-chinolyl]-6-methyl-2H-3,6-dihydro-1,3,4-thiadiazin-2-on;
(h) 3-[1-Methyl-4-piperidyloxycarbonyl]-5-[1,2,3,4-tetrahydro-1,-(3,4-dimethoxy-benzoyl)-4,4-dimethyl-6-chinolyl]-6-methyl-2H-3,6-dihydro-1,3,4-thiadiazin-2-on;
(i) 3-[1-Methyl-2-(N,N-dimethylamino)-ethoxycarbonyl]-5-[1,2,3,4tetrahydro-1-(3,4-dimethoxy-benzoyl)-4,4-dimethyl-6-chinolyl]-6-methyl-2H-3,6-dihydro-1,3,4-thiadiazin-2-on
gemäß Anspruch 1 oder ein Enantiomer der genannten Verbindungen.

4. Verfahren zur Herstellung von Verbindungen der Formel I nach Anspruch 1 sowie von deren Salzen, dadurch gekennzeichnet, daß man eine Verbindung der Formel II 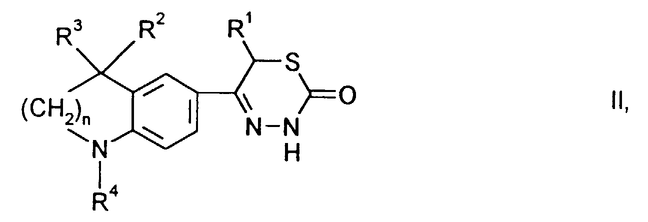 worin
R¹, R², R³, R⁴ und n die bereits angegebenen Bedeutungen haben,
mit einer Verbindung der Formel III
X-CO₂-Q-R⁵ III,
worin
R⁵ und Q die angegebenen Bedeutungen haben und
x Cl, Br, OH oder eine reaktionsfähige veresterte OH-Gruppe bedeutet,
umsetzt,
oder daß man eine Verbindung der Formel IV 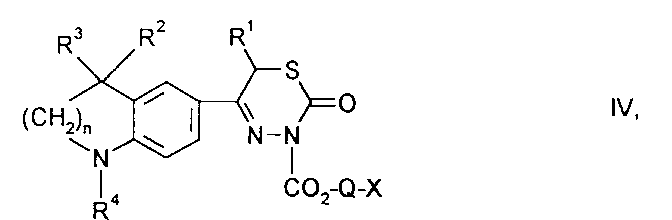 worin
R¹, R², R³, R⁴, Q, n und X die bereits angegebenen Bedeutungen besitzen,
mit einer Verbindung der Formel V
R⁵-H V,
worin
R⁵ die bereits angegebenen Bedeutungen hat,
umsetzt,
oder daß man eine Verbindung, die der Formel I entspricht, jedoch anstelle von R⁵ eine primäre oder eine sekundäre Aminogruppe besitzt, auf übliche Weise alkyliert,
oder daß man eine Verbindung, die an sich der Formel I entspricht, aber anstelle des Restes R⁴ ein Wasserstoffatom besitzt, auf übliche Weise acyliert,
oder daß man in einer Verbindung der Formel I einen Rest R⁴ in einen anderen Rest R⁴ umwandelt,
und/oder daß man eine Base der Formel I durch Behandeln mit einer Säure in eines ihrer Salze umwandelt.

5. Verfahren zur Herstellung pharmazeutischer Zubereitungen, dadurch gekennzeichnet, daß man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

6. Pharmazeutische Zubereitung, gekennzeichnet durch einen Gehalt an mindestens einer Verbindung der Formel I nach Anspruch 1 und/oder einem ihrer physiologisch unbedenklichen Salze.

7. Verwendung einer Verbindung der Formel I nach Anspruch 1 und/ oder eines ihrer physiologisch unbedenklichen Salze zur Herstellung eines Arzneimittels zur Bekämpfung von Krankheiten.

## Claims

1. 3-Alkoxycarbonylthiadiazinones of the formula I 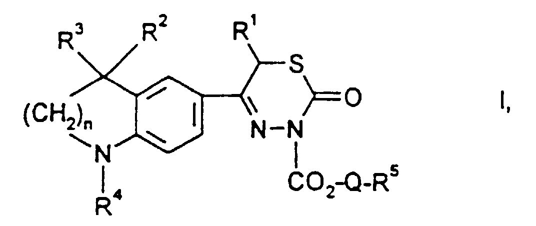 in which
R¹, R² and R³ in each case independently of one another are H or A,
R⁴ is acyl having 1 to 15 C atoms,
R⁵ is NH, NHA, NA₂ or a saturated, three- to eight-membered heterocyclic radical having at least one N atom, which can be substituted by A and in which additionally a further CH₂ group in the ring can be replaced by an NH or an NA group, an O or an S atom,
Q is absent or is branched or unbranched alkylene having 1 to 10 C atoms,
n is 1, 2 or 3 and
A is alkyl having 1 to 6 C atoms,
and their physiologically acceptable salts.

2. An enantiomer of a compound of the formula I according to Claim 1.

3. (a) 3-[2-(N,N-Diethylamino)ethoxycarbonyl]-5-[1,2,3,4-tetrahydro-1-(3,4-dimethoxybenzoyl)-6-quinolyl]-6-methyl-2H-3,6-dihydro-1,3,4-thiadiazin-2-one;
(b) 3-[3-(N,N-diethylamino)propoxycarbonyl]-5-[1,2,3,4-tetrahydro-1-(3,4-dimethoxybenzoyl)-6-quinolyl]-6-methyl-2H-3,6-dihydro-1,3,4-thiadiazin-2-one;
(c) 3-[6-(N,N-dimethylamino)hexyloxycarbonyl]-5-[1,2,3,4-tetrahydro-1-(3,4-dimethoxybenzoyl)-6-quinolyl]-6-methyl-2H-3,6-dihydro-1,3,4-thiadiazin-2-one;
(d) 3-[1-methyl-2-(N,N-dimethylamino)ethoxycarbonyl]-5-[1,2,3,4-tetrahydro-1-(3,4-dimethoxybenzoyl)-6-quinolyl]-6-methyl-2H-3,6-dihydro-1,3,4-thiadiazin-2-one;
(e) 3-[1-methyl-4-piperidyloxycarbonyl]-5-[1,2,3,4-tetrahydro-1-(3,4-dimethoxybenzoyl)-6-quinolyl]-6-methyl-2H-3,6-dihydro-1,3,4-thiadiazin-2-one;
(f) 3-[2-(N,N-diethylamino)ethoxycarbonyl]-5-[1,2,3,4-tetrahydro-1-(3,4-dimethoxybenzoyl)-4,4-dimethyl-6-quinolyl]-6-methyl-2H-3,6-dihydro-1,3,4-thiadiazin-2-one;
(g) 3-[6-(N,N-dimethylamino)hexyloxycarbonyl]-5-[1,2,3,4-tetrahydro-1-(3,4-dimethoxybenzoyl)-4,4-dimethyl-6-quinolyl]-6-methyl-2H-3,6-dihydro-1,3,4-thiadiazin-2-one;
(h) 3-[1-(methyl-4-piperidyloxycarbonyl]-5-[1,2,3,4-tetrahydro-1-(3,4-dimethoxybenzoyl)-4,4-dimethyl-6-quinolyl]-6-methyl-2H-3,6-dihydro-1,3,4-thiadiazin-2-one;
(i) 3-[1-(methyl-2-(N,N-dimethylamino)ethoxycarbonyl]-5-[1,2,3,4-tetrahydro-1-(3,4-dimethoxybenzoyl)-4,4-dimethyl-6-quinolyl]-6-methyl-2H-3,6-dihydro-1,3,4-thiadiazin-2-one
according to Claim 1 or an enantiomer of the compounds mentioned.

4. Process for the preparation of compounds of the formula I according to Claim 1, and of their salts, characterized in that a compound of the formula II 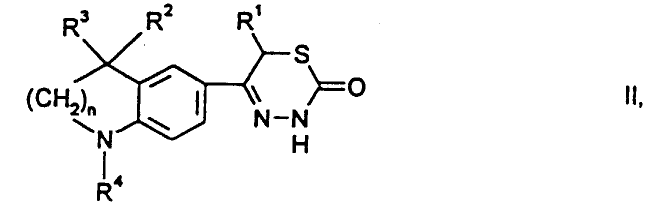 in which
R¹, R², R³, R⁴ and n have the meanings already indicated, is reacted with a compound of the formula III
**X-CO**_{**2**}**-Q-R**^{**5**} **III**
in which
R⁵ and Q have the meanings indicated and
X is Cl, Br, OH or a reactive esterified OH group,
or in that a compound of the formula IV 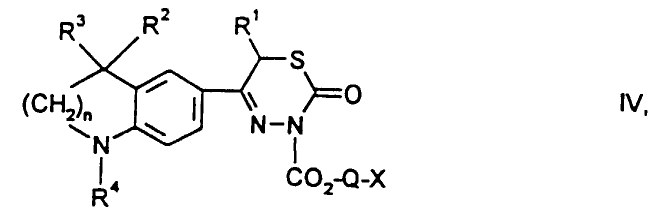 in which
R¹, R², R³, R⁴, Q, n and X have the meanings already indicated, is reacted with a compound of the formula V
**R**^{**5**}**-H V**
in which
R⁵ has the meanings already indicated,
or in that a compound which corresponds to the formula I, but instead of R⁵ has a primary or a secondary amino group, is alkylated in a customary manner,
or in that a compound which corresponds to the formula I, but instead of the radical R⁴ has a hydrogen atom, is acylated in a customary manner,
or in that in a compound of the formula I, a radical R⁴ is converted into another radical R⁴,
and/or in that a base of the formula I is converted into one of its salts by treating with an acid.

5. Process for the production of pharmaceutical preparations, characterized in that a compound of the formula I according to Claim 1 and/or one of its physiologically acceptable salts is brought into a suitable dose form together with at least one solid, liquid or semi-solid excipient or auxiliary.

6. Pharmaceutical preparation, characterized in that it contains at least one compound of the formula I according to Claim 1 and/or one of its physiologically acceptable salts.

7. Use of a compound of the formula I according to Claim 1 and/or one of its physiologically acceptable salts for the production of a medicament for controlling diseases.

## Revendications

1. 3-Alcoxycarbonylthiadiazinones de formule I 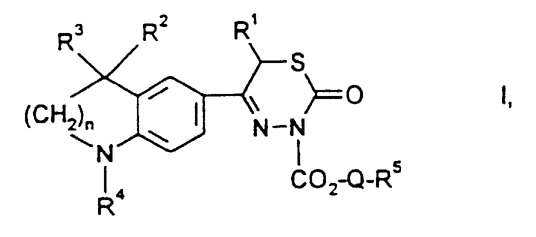 dans laquelle
R¹, R² et R³ représentent chacun, indépendamment les uns des autres, H ou A,
R⁴ un acyle ayant de 1 à 15 atomes C,
R⁵ NH₂, NHA, NA₂ ou un radical hétérocyclique saturé à trois à huit chaînons ayant au moins un atome N, qui peut être substitué par A et dans lequel, en outre, un groupe CH₂ supplémentaire du noyau peut être remplacé par un groupe NH, NA, un atome O ou un atome F,
Q est absent ou représente des alkyles ramifiés ou non ayant 1 à 10 atomes C,
n vaut 1, 2 ou 3 et
A représente un alkyle ayant 1 à 6 atomes C,
ainsi que leurs sels physiologiquement acceptables.

2. Enantiomère d'un composé de formule I selon la revendication 1.

3. (a) 3-[2-(N,N-diéthylamino)-éthoxycarbonyl]-5-[1,2,3,4-tétrahydro-1-(3,4-diméthoxybenzoyl)-6-chinolyl]-6-méthyl-2H-3,6-dihydro-1,3,4-thiadiazin-2-one;
(b) 3-[3-(N,N-diéthylamino)-propoxycarbonyl]-5-[1,2,3,4-tétrahydro-1-(3,4-diméthoxybenzoyl)-6-chinolyl]-6-méthyl-2H-3,6-dihydro-1,3,4-thiadiazin-2-one;
(c) 3-[6-(N,N-diméthylamino)-hexyloxy-carbonyl]-5-[1,2,3,4-tétrahydro-1-(3,4-diméthoxybenzoyl)-6-chinolyl]-6-méthyl-2H-3,6-dihydro-1,3,4-thiadiazin-2-one;
(d) 3-[1-méthyl-2-(N,N-diméthylamino)-éthoxycarbonyl]-5-[1,2,3,4-tétrahydro-1-(3,4-diméthoxybenzoyl)-6-chinolyl]-6-méthyl-2H-3,6-dihydro-1,3,4-thiadiazin-2-one;
(e) 3-[1-méthyl-4-pipéridyloxycarbonyl]-5-[1,2,3,4-tétrahydro-1-(3,4-diméthoxybenzoyl)-6-chinolyl]-6-méthyl-2H-3,6-dihydro-1,3,4-thiadiazin-2-one;
(f) 3-[2-(N,N-diéthylamino)-éthoxycarbonyl]-5-[1,2,3,4-tétrahydro-1-(3,4-diméthoxybenzoyl)-4,4-diméthyl-6-chinolyl]-6-méthyl-2H-3,6-dihydro-1,3,4-thiadiazin-2-one;
(g) 3-[6-(N,N-diméthylamino)-hexyloxycarbonyl]-5-[1,2,3,4-tétrahydro1-(3,4-diméthoxy-benzoyl)-4,4-diméthyl-6-chinolyl]-6-méthyl-2H-3,6-dihydro-1,3,4-thiadiazin-2-one;
(h) 3-[1-méthyl-4-pipéridyloxycarbonyl]-5-[1,2,3,4-tétrahydro-1-(3,4-diméthoxybenzoyl)-4,4-diméthyl-6-chinolyl]-6-méthyl-2H-3,6-dihydro-1,3,4-thiadiazin-2-one;
(i) 3-[1-méthyl-2(N,N-diméthylamino)éthoxycarbonyl]-5-[1,2,3,4-tétrahydro-1-(3,4-diméthoxybenzoyl)-4,4-diméthyl-6-chinolyl]-6-méthyl2H-3,6-dihydro-1,3,4-thiadiazin-2-one;
selon la revendication 1 ou énantiomère des composés mentionnés.

4. Procédé de préparation de composés de formule I selon la revendication 1 ainsi que de leurs sels, caractérisé en ce que l'on fait réagir un composé de formule II 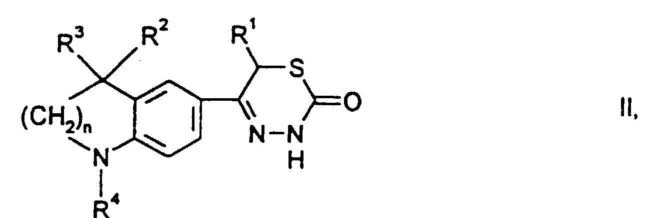 dans laquelle R¹, R², R³, R⁴ et n ont les significations déjà indiquées,
avec un composé de formule III
X-CO₂-Q-R⁵ III
dans laquelle
R⁵ et Q ont les significations indiquées et
X représente CI, Br, OH ou un groupe OH estérifié réactif,
ou en ce que l'on fait réagir un composé de formule IV 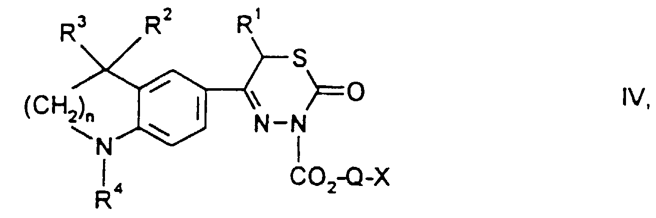 dans laquelle
R¹, R², R³, R⁴, Q, n et X ont les significations déjà indiquées,
avec un composé de formule V
R⁵-H **V,**
dans laquelle
R⁵ a les significations déjà indiquées,
ou en ce que l'on alkyle, de manière usuelle, un composé qui correspond à la formule I mais possède un groupe amino primaire ou secondaire à la place de R⁵, ou en ce que l'on acyle, de manière usuelle, un composé qui correspond à la formule I mais possède un atome d'hydrogène à la place du radical R⁴,
ou en ce que l'on transforme dans un composé de formule I, un radical R⁴ en un autre radical R⁴,
et/ou en ce que l'on transforme une base de formule I en l'un de ses sels par traitement avec un acide.

5. Procédé de préparation de compositions pharmaceutiques, caractérisé en ce que l'on réunit un composé de formule I selon la revendication 1 et/ou l'un de ses sels physiologiquement acceptables avec au moins un support ou additif solide, liquide ou semi-liquide dans une forme de dosage appropriée.

6. Composition pharmaceutique, caractérisée en ce qu'elle contient au moins un composé de formule I selon la revendication 1 et/ou l'un de ses sels physiologiquement acceptables.

7. Utilisation d'un composé de formule I selon la revendication 1, et/ou de l'un de ses sels physiologiquement acceptables pour la préparation d'un médicament en vue de la lutte contre des maladies.
